# FASCICULE DE BREVET EUROPEEN

(11) **EP 0 398 783 B1**
(45) Date de publication et mention de la délivrance du brevet: **13.07.1994**
(21) Numéro de dépôt: 90401223.4
(22) Date de dépôt: 09.05.1990
(51) Int. Cl.: C07C 255/40, C07C 255/17, C07C 255/31, C07C 253/14

(54) **Procédé de synthèse de cyanures d'acyle**
Verfahren zur Herstellung von Acylcyaniden
Process for the synthesis of acyl cyanides

(30) Priorité: 19.05.1989 FR 8906564
(43) Date de publication de la demande: 22.11.1990
(73) Titulaire: ELF ATOCHEM S.A., 92800 Puteaux (FR)
(72) Inventeur: Devic, Michel, F-69110 Saint Foy Les Lyon (FR); Tellier, Pierre, F-69110 Saint Foy Les Lyon (FR)

(56) Documents cités:
- EP-A- 0 038 987
- FR-A- 2 346 323
- FR-A- 2 353 524
- FR-A- 2 364 894

## Description

### PROCEDE DE SYNTHESE DE CYANURES D'ACYLE

La présente invention concerne un procédé de synthèse de cyanures d'acyle. Ce procédé consiste à faire réagir des halogénures d'acide avec un cyanure alcalin. Les cyanures d'acyle sont des intermédiaires de synthèse organique par exemple, des herbicides.

Le brevet FR 2 353 524 décrit une synthèse de cyanure de benzoyle C₆H₅COCN par réaction du chlorure de benzoyle avec du cyanure de sodium en excès molaire en présence de nitrile d'acide carboxylique et de cyanure de cuivre. Le brevet FR 2 346 323 décrit une réaction similaire mais beaucoup plus générale puisqu'elle s'applique à toute une famille de cyanures d'acyle et consiste à faire réagir du cyanure de sodium avec un halogénure d'acide en excès en présence de cyanure de cuivre ou de zinc. Ces procédés ont l'inconvénient de nécessiter la présence de métaux lourds et obligent donc à des traitements compliqués pour éviter leur présence dans les effluents. On trouve dans TETRAHEDRON LETTERS (Pergamon Press) n° 26, pages 2275-2278 (1974) un procédé limité à la synthèse du cyanure de benzoyle par action du cyanure de sodium sur le chlorure de benzoyle en solution dans le chlorure de méthylène et en présence de bromure de tétrabutylammonium, le rendement en C₆H₅COCN sur C₆H₅COCl ne dépasse pas 60 %. Le brevet FR 2 364 894 décrit la synthèse de C₆H₅COCN par action de C₆H₅COCl sur le NaCN dans un solvant en présence d'anhydride benzoïque (C₆H₅CO-O-CO-C₆H₅) ou de produits qui peuvent générer de l'anhydride benzoïque dans les conditions de la réaction. On a reproduit l'exemple 1 de ce brevet, c'est-à-dire la réaction de chlorure de benzoyle, d'anhydride benzoïque et de cyanure de sodium dans le xylène entre 140 et 145°C pendant 8 heures, et on a trouvé un rendement molaire en cyanure de benzoyle sur l'ensemble anhydride benzoïque/chlorure de benzoyle engagé de 51,2 % et un taux de conversion du chlorure de benzoyle engagé de 60,5 %. On a recommencé, cette fois en utilisant du cyanure de sodium humide (0,4 g d'eau pour 36,75 g de NaCN), le rendement est monté de 51,2 à 88,2 %.

On a reproduit aussi l'exemple 2, c'est-à-dire la réaction du chlorure de benzoyle, du cyanure de sodium et du benzoate de sodium dans le xylène à 135°C. Le benzoate est présenté comme devant générer, par action sur le chlorure de benzoyle, de l'anhydride benzoïque. On a trouvé un taux de conversion du chlorure de benzoyle engagé de 23 % et un rendement molaire de 14,9 % en cyanure de benzoyle par rapport au chlorure de benzoyle engagé, au lieu de 94 % annoncé. On a recommencé cet exemple 2 mais en utilisant cette fois du cyanure de sodium humide (0,5 g d'eau pour 29,4 g de NaCN) ; le taux de conversion est passé de 23 à 85,7 % et le rendement de 14,9 à 67,8 %.

La présence d'eau est donc nécessaire pour obtenir un rendement économiquement acceptable selon FR 2 364 894.

L'exemple 3 de FR 2 364 894 concerne la réaction du chlorure de benzoyle, du cyanure de sodium et d'eau dans le xylène à 135°C.

C'est similaire à l'exemple 2 mais le benzoate comme précurseur d'anhydride est remplacé par l'eau. On a reproduit cet exemple 3, et après 2 heures de chauffage à 135°C le rendement ne dépasse pas 60 %.

On a maintenant trouvé un nouveau procédé pour préparer les cyanures d'acyle qui donne de meilleurs rendements à plus basse température, ce qui diminue fortement les sous produits de réaction et qui est reproductible. La présente invention est donc un procédé de synthèse de cyanure d'acyle de formule (I) : 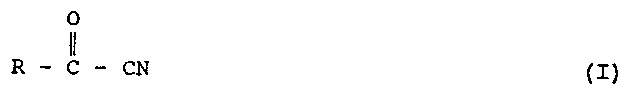
dans laquelle R est soit un radical alkyle ayant de 1 à 8 atomes de carbone, soit un radical cycloalkyle ayant de 3 à 12 atomes de carbone, soit un radical aryle, soit un reste hétérocyclique pouvant être condensé avec un noyau benzénique, tous ces radicaux R étant éventuellement substitués, consistant à faire réagir des halogénures d'acide de formule (II) : 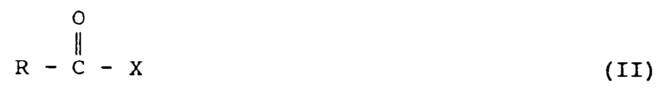
dans laquelle R a la définition précédente et X désigne un halogène, avec des cyanures alcalins, caractérisé en ce que la réaction a lieu en présence d'un produit contenant des motifs oxyde d'alkylène et en présence de quantités catalytiques d'eau.

La réaction de l'invention se fait selon la formule : 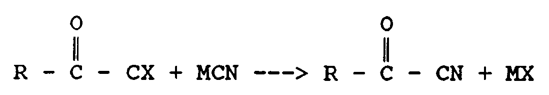
dans laquelle M désigne un métal alcalin et est avantageusement conduite dans un solvant. Après réaction, l'halogénure alcalin et l'excès éventuel de cyanure alcalin sont éliminés par filtration et lavage avec du solvant. Le cyanure d'acyle pur est obtenu par distillation du milieu réactionnel filtré.

Les halogénures d'acide utilisés comme matières premières sont définis par la formule (II). Dans cette formule R désigne de préférence un radical alkyle linéaire ou ramifié ayant de 1 à 4 carbones et pouvant être substitués ; R désigne aussi, de préférence, un radical cycloalkyle ayant 5 ou 6 carbones et pouvant être substitué ; R désigne aussi, de préférence, un radical phényle ou naphtyle et pouvant être substitué ; R désigne aussi, de préférence, des restes hétérocycliques pentagonaux ou hexagonaux, le cas échéant substitués

Dans la formule (II), X désigne avantageusement du chlore ou du brome.

L'halogénure d'acide utilisé est en général ajouté peu à peu au mélange réactionnel sous agitation, sous forme pure ou dilué, par le solvant de la réaction. La durée de l'addition peut varier de quelques minutes à plusieurs heures.

La durée préférée est d'environ 1 heure.

Le cyanure alcalin, de préférence de sodium ou potassium, est utilisé en quantité stoéchiométrique ou bien en excès, à raison de 1 à 2 moles par mole d'halogénure d'acide, la quantité préférée est de 1 à 1,25 mole par mole d'halogénure d'acide.

La réaction a lieu avantageusement en présence d'un solvant inerte.

On peut utiliser tous les solvants ne réagissant pas dans les conditions de la réaction avec l'halogénure d'acide, ou bien avec le cyanure alcalin.

Comme solvant convenant à la réaction on peut citer :
- les hydrocarbures benzéniques tels que le benzène, le toluène, le xylène, le chlorobenzène, etc...
- les hydrocarbures aliphatiques halogénés tels que le trichloroéthylène, le tétrachloroéthane,
- les éthers ou esters, inertes dans les conditions de la réaction.

Les solvants préférés de la réaction sont le toluène et le xylène.

La quantité de solvant peut varier dans de larges limites. Avantageusement, il suffit de 150 à 500 ml par mole d'halogénure d'acide.

On ne sortirait pas du cadre de l'invention en utilisant une quantité plus importante de solvant, mais on serait obligé de distiller une quantité plus importante pour récupérer le cyanure d'acyle.

La réaction peut s'effectuer à des températures comprises entre 60 et 150°C. La température préférée étant comprise entre 90 et 120°C.

La réaction s'effectue avantageusement à pression atmosphérique si le solvant choisi le permet. La réaction peut aussi s'effectuer sous pression de gaz inerte, ou sous pression de vapeur de solvant lorsque le point d'ébullition du solvant choisi est inférieur à la température de réaction.

La réaction est très rapide et est, par exemple, achevée après 15 à 30 minutes à 120°C. Le chauffage est cependant maintenu pendant une durée de 1 à 2 heures pour éliminer toute trace d'halogénure d'acide. La durée de réaction préférée est de 2 heures à 95°C.

Le produit contenant des motifs oxyde d'alkylène contient Avantageusement, il entre 2 et 200 motifs choisis parmi l'oxyde d'éthylène et l'oxyde de propylène.

C'est par exemple un produit contenant une ou plusieurs chaînes :

O-(CH₂-CH₂-O)ₙ-CH₂-CH₂-OH

le nombre total du ou des n étant compris entre 2 et 200, on peut citer aussi :
- les alkylphénolpolyoxyéthylènés: 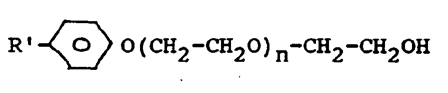 ou R′ est un alkyle ayant jusqu'à 20 carbones, R′ étant par exemple C₈H₁₇ ou C₉H₁₉ ou C₁₂H₁₇, n étant compris entre 20 et 100.
- les stéarates polyoxyéthylènés :

   CH₃-(CH₂)₁₆-COO-(CH₂-CH₂O)ₙ -CH₂-CH₂-OH
- les polyéthylèneglycol simples de formule :

   OH-(CH₂-CH₂O)ₙ-CH₂-CH₂-OH

   de masse moléculaire comprise entre 100 et 4000.

Parmi les agents ayant plusieurs chaînes polyéthylènées, on peut citer les dérivés triglycérides des polyéthylèneglycols dont le nombre total de motifs oxyde d'éthylène est compris entre 20 et 150.

On peut utiliser les produits similaires aux produits ci-dessus dans lesquels le motif oxyde d'éthylène est remplacé par le motif oxyde de propylène ou un mélange de motifs oxyde d'éthylèneoxyde de propylène.

La quantité de ce produit peut varier de 0,1 à 10 g par mole d'halogénure d'acide. La quantité préférée étant de 0,4 à 2 g.

Le produit est généralement ajouté dans le solvant de la réaction mais il peut aussi être ajouté tout ou en partie avec l'halogénure d'acide pur ou dilué avec du solvant. De faibles quantités d'eau doivent être ajoutées aux réactifs pour obtenir un taux de conversion et un rendement élevé.

La quantité d'eau qui doit être présente au cours de la réaction est comprise entre 0,2 et 2 g d'eau par mole d'halogénure d'acide. La quantité préférée d'eau est de 0,5 à 1 g d'eau par mole d'halogénure d'acide.

Le mode d'introduction de l'eau doit assurer une bonne répartition de celle-ci sur les réactifs.

Dans les exemples suivants le taux de conversion exprime la quantité de l'halogénure d'acide disparue par rapport à la quantité initiale et le rendement exprime le rapport entre le nombre de moles de cyanure d'acyle obtenu et le nombre de moles d'halogénure d'acide initiales.

### EXEMPLE 1

Dans un réacteur en verre, muni d'une agitation et d'un réfrigérant contenant 150 cm³ de xylène et 0,5 g de nonylphénol polyoxyéthyléné de formule : 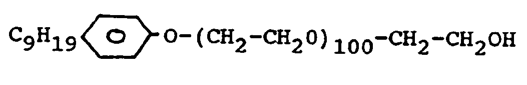
(commercialisé par la Société GAF sous le nom d'ANTAROX CO 990), on ajoute 36,75 g de cyanure de sodium anhydre (0,75 M) et 0,4 g d'eau.

Puis on coule en 0,5 heure à 125°C 70,3 g de chlorure de benzoyle (0,50 M), on porte la température à 140°C pendant 2 heures.

Après refroidissement, on filtre et on lave le précipité minéral (42,9 g).

Le filtrat est distillé et l'on recueille 60,05 g de cyanure de benzoyle pur, soit un rendement de 91,6 %.

### EXEMPLE 2

Dans un réacteur en verre muni d'une agitation et d'un réfrigérant contenant :
150 cm³ de xylène et
0,5 g d'ANTAROX CO 990,
on ajoute 24,5 g de cyanure de sodium (0,5 M) et 0,5 g d'eau. On chauffe à 95°C et on coule en 1 heure 70,3 g de chlorure de benzoyle (0,5 M). On maintient la température à 95°C pendant deux heures puis, après refroidissement, on filtre et on lave avec du xylène (précipité minéral de 29,2 g).

On obtient, après filtration, une solution de xylène d'une masse de 231,4 g. Par analyse par chromatographie gazeuse avec étalon interne, on dose 26,3 % de cyanure de benzoyle, soit une quantité totale de 60,85 g de cyanure de benzoyle pur, correspondant à un rendement de 92,8 %.

### EXEMPLE 3

On procède comme pour l'exemple 2 avec :
150 cm³ de xylène,
0,5 g d'ANTAROX CO 990,
29,4 g de cyanure de sodium (0,6 M) anhydre,
0,4 g d'eau,
70,3 g de chlorure de benzoyle (0,5 M),
on obtient, après réaction, un taux de conversion du chlorure de benzoyle de 99 % et un rendement par rapport au chlorure de benzoyle engagé s'élevant à 86,2 %.

### EXEMPLE 4 (non conforme à l'invention)

En procédant exactement de la même façon qu'à l'exemple 3, mais en omettant d'ajouter l'eau, on obtient un taux de transformation de 32 % et un rendement chimique de 23,8 %.

### EXEMPLE 5 (non conforme à l'invention et semblable à l'exemple 3 de FR 2 364 894)

En procédant exactement de la même façon qu'à l'exemple 3, mais en omettant d'ajouter le dérivé polyoxyéthylène (ANTAROX CO 990), on obtient un taux de conversion de 79 % et un rendement de 70,2 %.

### EXEMPLE 6

On procède exactement comme pour l'exemple 3 mais on remplace l'ANTAROX CO 990 par de l'ANTAROX CO 850 de formule :

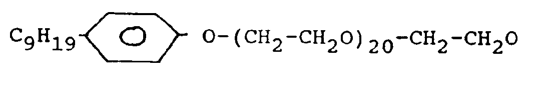

On obtient un taux de conversion de 99 % et un rendement de 85,1 %.

### EXEMPLE 7

On procède exactement comme pour l'exemple 3 mais en remplaçant l'ANTAROX CO 990 par un stéarate polyoxyéthylé de 100 motifs oxyde d'éthylène, commercialisé par la Société ICI sous le nom de BRI J 700 de formule :

CH₃-(CH₂)₁₆-COO-(CH₂-CH₂O)₁₀₀-CH₂-CH₂OH

On obtient un taux de conversion de 99 % et un rendement de 84,6 %.

### EXEMPLE 8

On procède exactement comme pour l'exemple 3 mais en remplaçant l'ANTAROX CO 990 par un triglycéride du polyoxyéthylèneglycol de 150 motifs oxyde d'éthylène, commercialisé par la Société ATLAS sous le nom de G 1295.

Le taux de conversion s'élève à 98,8 % et le rendement est de 83,3 %.

### EXEMPLE 9

On procède exactement comme pour l'exemple 3 mais en remplaçant l'ANTAROX CO 990 par un monostéarate mixte polyoxyéthylène et polyoxypropylène (ATLAS G 2162) contenant 25 motifs oxyéthylène, on obtient un taux de conversion de 100 % et un rendement de 84 %.

### EXEMPLE 10

On procède comme pour l'exemple 7 mais au lieu de filtrer après réaction, on distille le mélange brut contenant les sels minéraux.

On obtient un résidu de sels minéraux et de produits organiques pesant 43 g et 206 g de distillat contenant 55,4 g de cyanure de benzoyle, soit un rendement de 84,5 %.

### EXEMPLE 11

On procède exactement comme dans l'exemple 3 mais en remplaçant le xylène par un volume égal de toluène.

On obtient un taux de conversion de 98,6 % et un rendement de 83,2 %.

### EXEMPLE 12

On procède exactement comme pour l'exemple 3 mais en effectuant la coulée et le chauffage à une température de 105°C au lieu de 95°c, on obtient un taux de conversion de 99,1 % et un rendement de 83,8 %.

### EXEMPLE 13

Dans un ballon de 250 ml, on introduit :
14,7 g de cyanure de sodium,
0,2 g d'eau (0,011 mole),
80 g de xylène,
0,2 g d'ANTAROX 990.

On coule en une heure à 95°C 35,15 g de chlorure de benzoyle. On laisse réagir encore 3 heures à 98°C.

Le précipité est éliminé par filtration et lavage au xylène. On recueille ainsi 15,4 g de sel et 164 g de solution organique.

Le dosage par chromatographie en phase vapeur de la solution organique montre qu'elle contient 28,7 g de cyanure de benzoyle, ce qui représente un rendement de 87,6 % par rapport au chlorure de benzoyle engagé. Le taux de transformation du chlorure de benzoyle est de 99 %.

### EXEMPLE 14 (COMPARATIF)

On opère comme dans l'exemple 13 en remplaçant l'eau par 2,5 g d'anhydride benzoïque (0,011 mole).

Le taux de transformation du chlorure de benzoyle n'est que de 19 % et le rendement en cyanure de benzoyle par rapport au chlorure de benzoyle engagé n'est que de 9,7 %.

### EXEMPLE 15

On opère comme dans l'exemple 13 en remplaçant l'ANTAROX par 0,2 g de polyéthylène glycol de masse moyenne 200. Après introduction du chlorure de benzoyle, on maintient le milieu pendant 2 heures à 98°C et 2 heures à 115°C. La solution obtenue après séparation du sel contient 29,3 g de cyanure de benzoyle, ce qui correspond à un rendement de 89,5 % par rapport au chlorure de benzoyle engagé. Le taux de transformation du chlorure de benzoyle est de 98 %.

### EXEMPLE 16

On opère comme dans l'exemple 15 en employant un polyéthylène glycol de masse moyenne 3400. Le taux de transformation du chlorure de benzoyle est de 100 % et le rendement en cyanure de benzoyle de 91,2 % par rapport au chlorure de benzoyle engagé.

### EXEMPLE 17 (portant sur une plus grande quantité)

On opère comme dans l'exemple A en multipliant toutes les quantités par 8. Après introduction du chlorure de benzoyle, on maintient le milieu pendant encore 2 heures à 98°C et 2 heures à 115°C.

Après évaporation du sel, la solution est distillée. On recueille 234,4 g de cyanure de benzoyle, ce qui représente un rendement de 89,4 % par rapport au chlorure de benzoyle engagé. Le taux de transformation est de 100 %.

## Revendications

1. Procédé de synthèse de cyanures d'acyle de formule (I) : 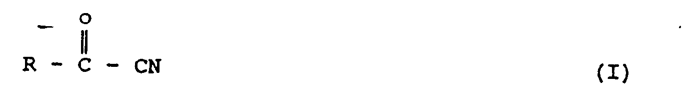 dans laquelle R est soit un radical alkyle ayant de 1 à 8 atomes de carbone, soit un radical cycloalkyle ayant de 3 à 12 atomes de carbone, soit un radical aryle, soit un reste hétérocyclique pouvant être condensé avec un noyau benzénique, tous ces radicaux R étant éventuellement substitués, consistant à faire réagir des halogénures d'acide de formule (II) : 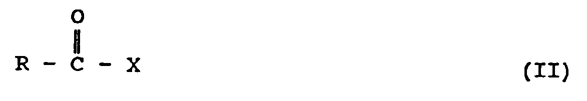 dans laquelle R a la définition précédente et X désigne un halogène, avec des cyanures alcalins, en présence de quantité5 catalytiques d'eau , caractérisé en ce que la réaction a lieu en présence d'un produit contenant des motifs oxyde d'alkylène

2. Procédé selon la revendication 1 caractérisé en ce qu'on opère en présence d'un solvant.

3. Procédé selon la revendication 1 ou 2, caractérisé en ce que le cyanure alcalin est utilisé à raison de 1 à 2 moles par mole d'halogénure d'acide et de préférence 1 à 1,25 mole.

4. Procédé selon l'une des revendications 1 à 3, caractérisé en ce qu'on utilise de préférence le cyanure de sodium.

5. Procédé selon l'une des revendications 1 à 4, caractérisé en ce qu'on utilise comme solvant de préférence le xylène ou le toluène.

6. Procédé selon l'une des revendications 1 à 5, caractérisé en ce que le produit contenant des motifs oxyde d'alkylène contient entre 2 et 200 motifs choisis parmi l'oxyde d'éthylène ou l'oxyde de propylène.

7. Procédé selon la revendication 6, caractérisé en ce que la quantité de produit contenant des motifs oxyde d'alkylène est comprise entre 0,1 et 10 g, et de préférence 0,4 à 2 g par mole d'halogénure d'acide.

8. Procédé selon l'une des revendications 1 à 7, caractérisé en ce que la quantité d'eau est comprise entre 0,2 et 2 g, et de préférence 0,5 à 1 g par mole d'halogénure d'acide.

9. Procédé selon l'une des revendications 1 à 8, caractérisé en ce que l'halogénure d'acide est du chlorure de benzoyle.

10. Procédé selon l'une des revendications 2 à 9, caractérisé en ce que la quantité de solvant est comprise entre 150 et 500 ml par mole d'halogénure d'acide.

## Patentansprüche

1. Verfahren zur Herstellung von Acylcyaniden der Formel (I) 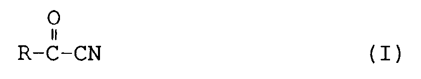 in der R ein Alkyl mit 1 bis 8 Kohlenstoffatomen, ein Cycloalkyl mit 3 bis 12 Kohlenstoffatomen, ein Arylrest, ein heterocyclischer Rest, gegebenenfalls mit kondensierten Benzolringen ist, wobei diese Reste R gegebenenfalls substiutiert sein können; bei der man Säurehalogenide der Formel (II) 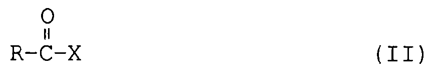 in der R der obigen Definition entspricht und X ein Halogen bezeichnet, mit Alkalicyaniden in Gegenwart von katalytischen Mengen Wasser reagieren läßt, dadurch gekennzeichnet, daß die Reaktion in Gegenwart eines Produktes, das Alkylenoxideinheiten beinhaltet, durchgeführt wird.

2. Verfahren nach Anspruch 1, dadurch gekennzeichnet, daß man in Gegenwart eines Lösemittels arbeitet.

3. Verfahren nach Anspruch 1 oder 2, dadurch gekennzeichnet, daß 1 bis 2 Mol, vorzugsweise 1 bis 1,25 Mol Alkalicyanid pro Mol Säurehalogenid verwendet werden.

4. Verfahren nach einem der Ansprüche 1 bis 3, dadurch gekennzeichnet, daß man vorzugsweise Natriumcyanid verwendet.

5. Verfahren nach einem der Ansprüche 1 bis 4, dadurch gekennzeichnet, daß man als Lösemittel vorzugsweise Xylol oder Toluol verwendet.

6. Verfahren nach einem der Ansprüche 1 bis 5, dadurch gekennzeichnet, daß das Produkt, das Alkyleneinheiten beinhaltet, zwischen 2 bis 200 Einheiten enthält, die aus der Gruppe von Ethylenoxid oder Propylenoxid sind.

7. Verfahren nach Anspruch 6, dadurch gekennzeichnet, daß die Menge an Produkt, das Alkylenoxideinheiten enthält, zwischen 0,1 und 10 g, vorzugsweise zwischen 0,4 und 2 g pro Mol Säurehalogenid ist.

8. Verfahren nach einem der Ansprüche 1 bis 7, dadurch gekennzeichnet, daß die Menge an Wasser zwischen 0,2 und 2 g, vorzugsweise zwischen 0,5 und 1 g pro Mol Säurehalogenid ist.

9. Verfahren nach einem der Ansprüche 1 bis 8, dadurch gekennzeichnet, daß das Säurehalogenid Benzoylchlorid ist.

10. Verfahren nach einem der Ansprüche 1 bis 9, dadurch gekennzeichnet, daß die Menge an Lösemittel zwischen 150 und 500 ml pro Mol Säurehalogenid liegt.

## Claims

1. Process for the synthesis of acyl cyanides of formula (I) : 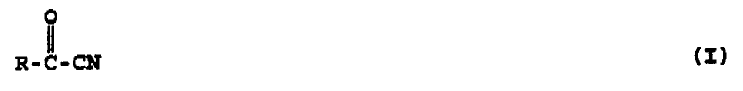 in which R is either an alkyl radical containing from 1 to 8 carbon atoms or a cycloalkyl radical containing from 3 to 12 carbon atoms, or an aryl radical, or a heterocyclic residue which may be condensed with a benzene nucleus, all these radicals R being optionally substituted, consisting in reacting acid halides of formula (II) : 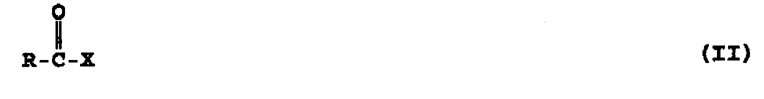 in which R has the above definition and X denotes a halogen, with alkali metal cyanides, in the presence of catalytic quantities of water characterized in that the reaction takes place in the presence of a product containing alkylene oxide units.

2. Process according to claim 1, characterized in that the operation is carried out in the presence of a solvent.

3. Process according to claim 1 or 2, characterized in that the alkali metal cyanide is employed in a proportion of 1 to 2 moles, and preferably 1 to 1.25 moles, per mole of acid halide.

4. Process according to one of claims 1 to 3, characterized in that sodium cyanide preferably employed.

5. Process according to one of claims 1 to 4, characterized in that xylene or toluene is preferably employed as solvent.

6. Process according to one of claims 1 to 5, characterized in that the product containing alkylene oxide units contains between 2 and 200 units chosen from ethylene oxide and propylene oxide.

7. Process according to claim 6, characterized in that the quantity of product containing alkylene oxide units is between 0.1 and 10 g, and preferably 0.4 to 2 g per mole of acid halide.

8. Process according to one of claims 1 to 7, characterized in that the quantity of water is between 0.2 and 2 g, and preferably 0.5 to to 1 g per mole of acid halide.

9. Process according to one of claims 1 to 8, characterized in that the acid halide is benzoyl chloride.

10. Process according to one of claims 2 to 9, characterized in that the quantity of solvent is between 150 and 500 ml per mole of acid halide.
